# EUROPÄISCHE PATENTANMELDUNG

(11) **EP 4 782 456 A1**
(43) Veröffentlichungstag der Anmeldung: **29.07.2026**
(21) Anmeldenummer: 25153737.9
(22) Anmeldetag: 24.01.2025
(51) Int. Cl.: C07F 9/6596, C07D 215/02, C07D 401/02, C07F 15/00

(54) **SYNTHESE UND ANWENDUNG LUFTSTABILER SAUERSTOFF-SUBSTITUIERTER BIARYL-PHOSPHASILINAN-LIGANDEN IN C X-KREUZKUPPLUNGSREAKTIONEN**

(71) Anmelder: Ludwig-Maximilians-Universität München, 80539 München (DE)
(72) Erfinder: TRAPP, Oliver, München (DE); BERTHOLD, Dino, München (DE); KREMSER, Christoph, München (DE); LAUER, Christoph, München (DE)
(74) Vertreter: Weickmann & Weickmann PartmbB

(57) **Zusammenfassung**

Die vorliegende Erfindung betrifft cyclische Biarylphosphine und deren Verwendung als Liganden in Palladiumkomplexen bei palladiumkatalysierten Kreuzkupplungsreaktionen wie z.B. der Buchwald-Hartwig-Kupplung und der Suzuki-Miyaura-Kreuzkupplung.

## Beschreibung

Die vorliegende Erfindung betrifft cyclische Biarylphosphine und deren Verwendung als Liganden in Metallkomplexen und insbesondere in Palladiumkomplexen bei palladiumkatalysierten Kreuzkupplungsreaktionen wie z.B. der Buchwald-Hartwig-Kupplung und der Suzuki-Miyaura-Kreuzkupplung.

Unter einer Kreuzkupplung wird eine Kupplungsreaktion verstanden, bei der zwei unterschiedliche Moleküle in Anwesenheit eines geeigneten Katalysators unter Ausbildung einer Kohlenstoff-Kohlenstoff- oder Kohlenstoff-Heteroatom-Bindung miteinander reagieren. Von großer praktischer Bedeutung (z.B. bei der Synthese pharmazeutischer Wirkstoffe) sind insbesondere palladiumkatalysierte Kreuzkupplungen. Beispielhaft können in diesem Zusammenhang die Stille-Kupplung, Suzuki-Kupplung (auch als Suzuki-Miyaura-Kupplung bekannt) oder Negishi-Kupplung genannt werden. Im Jahr 2010 erhielten R.F. Heck, E. Negishi und A. Suzuki den Nobelpreis für Chemie für ihre Arbeiten zu Kupplungsreaktionen.

In den folgenden Übersichtsartikeln wird die Verwendung von Kreuzkupplungsreaktionen bei der Synthese pharmazeutischer Wirkstoffe eingehender beschrieben:
- H.C. Shen, "Selected Applications of Transition Metal-Catalyzed Carbon-Carbon Cross-Coupling Reactions in the Pharmaceutical Industry", S. 25-96, in "Applications of Transition Metal Catalysis in Drug Discovery and Development: An Industrial Perspective", Ed.: M.L. Crawley and B.M. Trost, 2012, John Wiley & Sons;
- Q. Gu et al., "Palladium catalyzed C-C and C-N bond forming reactions: An update on the synthesis of pharmaceuticals from 2015-2020", Org. Chem. Front., 2021, 8, S. 384-414.

Eine weitere dem Fachmann bekannte Kupplungsreaktion ist die Buchwald-Hartwig-Kupplung, bei der ein Aryl- oder Heteroarylhalogenid oder -pseudohalogenid und ein primäres oder sekundäres Amin in Anwesenheit einer Base und eines palladiumhaltigen Katalysators unter Ausbildung einer C-N-Bindung miteinander umgesetzt werden.

In den folgenden Übersichtsartikeln wird der aktuelle Stand auf dem Gebiet der C-N-Kupplungsreaktionen zusammengefasst:
- R. Dorel et al., "The Buchwald-Hartwig Amination After 25 Years", Angew. Chem. Int. Ed., 2019, 58, S. 17118-17129;
- S.L. Buchwald et al., "Dialkylbiaryl phosphines in Pd-catalyzed amination: a user's guide", Chem. Sci., 2011, 2, S. 27-50;
- S.L. Buchwald et al., "Biaryl Phosphane Ligands in Palladium-Catalyzed Amination", Angew. Chem. Int. Ed., 2008, 47, S. 6338-6361;
- S.L. Buchwald et al., "Applications of Palladium-Catalyzed C-N Cross-Coupling Reactions", Chem. Rev., 2016 116, S. 12564-12649.

Übliche Katalysatoren auf dem Gebiet der Kupplungsreaktionen sind Palladiumkomplexe, die einen oder mehrere Phosphinliganden und optional weitere Liganden enthalten. Diese Pd-Komplexe mit geeigneten Phosphinliganden können vorab hergestellt und bis zu ihrer Verwendung gelagert oder alternativ *in-situ* während der zu katalysierenden Reaktion generiert werden (z.B. durch getrennte Zugabe eines Palladiumsalzes und des Phosphins zum Reaktionsmedium, so dass die Ausbildung eines phosphinhaltigen Pd-Komplexes erst im Reaktionsmedium erfolgt).

Es ist bekannt, dass nicht-cyclische Biarylphosphine als Liganden für Pd-Komplexe in palladiumkatalysierten Kupplungsreaktionen (wie z.B. der Buchwald-Hartwig-Kupplung) fungieren können, siehe z.B. S.L. Buchwald et al., 2011, supra. In diesen nicht-cyclischen Phosphinen liegt kein phosphorhaltiger Ring (d.h. kein Ring, der Phosphor als Ringatom enthält) vor.

Es sind auch cyclische Biarylphosphine (d.h. Phosphine, in denen das Phosphoratom eines der ringbildenden Atome ist) als Liganden für Pd-Komplexe in palladiumkatalysierten Kupplungsreaktionen bekannt.

S. Shekhar et al., ACS Catal., 2019, 9, S. 11691-11708, und S. Shekhar et al., ACS Catal., 2020, 10, S. 15008-15018, beschreiben Biarylphosphorinane und deren Verwendung als Pd-Komplexliganden für palladiumkatalysierte Kupplungsreaktionen.

C. Maumela et al., RSC Adv., 2021, 11, S. 26883-26891, beschreiben Biarylphosphane und Biarylphosphatrioxadamantane und deren Verwendung als Pd-Komplexliganden für palladiumkatalysierte Suzuki-Kupplungen.

WO 2012/009698 A1 beschreibt ein monocyclisches, bicyclisches oder tricyclisches Biarylphosphin, wobei das heterocyclische Ringsystem neben dem Phosphoratom noch vier Kohlenstoffatome und optional mindestens ein weiteres Ringatom, das aus Kohlenstoff, Sauerstoff, Stickstoff, Phosphor und Schwefel ausgewählt ist, enthält.

Die Ausbildung einer Kohlenstoff-Heteroatom-Bindung (insbesondere einer C-N-Bindung) über eine Kupplungsreaktion, insbesondere eine Buchwald-Hartwig-Kupplung, erfordert bei der Verwendung N-heterocyclischer Arylhalogenide üblicherweise lange Reaktionszeiten. Sofern keine effizienteren Katalysatoren zur Verfügung stehen, kann beispielsweise die Reaktionstemperatur erhöht werden, um kürzere Reaktionszeiten zu realisieren. Dies führt aber üblicherweise zu unerwünschten Nebenreaktionen, die die Ausbeute des Kupplungsprodukts reduzieren.

Eine Aufgabe der vorliegenden Erfindung ist die Bereitstellung geeigneter Phosphine, die als Liganden in Metallkomplexen, insbesondere als Liganden in Palladiumkomplexen bei palladiumkatalysierten Kreuzkupplungsreaktionen verwendbar sind. Insbesondere sollten diese Phosphine als Liganden in Palladiumkomplexen auch dann effiziente C-N-Kupplungsreaktionen (z.B. in Form einer Buchwald-Hartwig-Kupplung) ermöglichen, wenn N-heterocyclische Arylhalogenide oder -pseudohalogenide als Reaktanten eingesetzt werden.

WO 2023/088620 A1 beschreibt ein Verfahren zur Herstellung von luftstabilen Biaryl-Phosphasilinan-Liganden durch die teilweise Deprotonierung eines Phosphins und Umsetzung mit einer Dihalogenid-Verbindung. Des Weiteren wird die Anwendung von ausschließlich im Biaryl-Abschnitt Kohlenstoff-substituierten Phosphasilinan-Liganden in Pd-katalysierten Kreuzkupplungen, wie z. B. der Suzuki-Miyaura-Kreuzkupplung und der Buchwald-Hartwig-Kupplung beschrieben.

Eine Aufgabe der vorliegenden Erfindung ist die Beschreibung und Schützung von Phosphasilinan-Liganden, welche Sauerstoff-Substituenten in ihrem Biaryl-Abschnitt aufweisen. Hierdurch wird die Aufgabe der Herstellung von aktiveren Liganden für Metallkomplexe, z.B. für Palladiumkomplexe, insbesondere zur Anwendung als Liganden für Pd-katalysierten Kreuzkupplungen gelöst.

Ein erster Aspekt der Erfindung ist ein Phosphin der Formel (1) 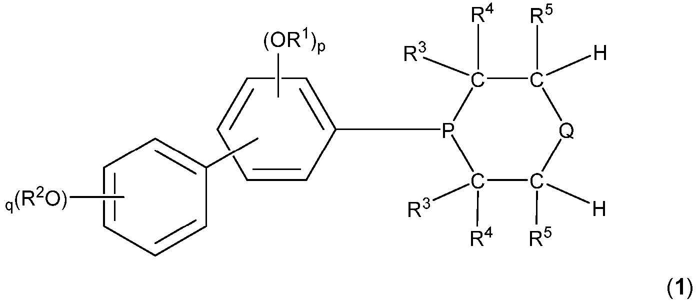 wobei
R¹ jeweils unabhängig Alkyl oder Aryl ist;
R² jeweils unabhängig Alkyl oder Aryl ist;
p 0, 1, 2, 3 oder 4 ist,
q 1, 2, 3, 4, oder 5 ist,
R³, R⁴ und R⁵ jeweils unabhängig voneinander Wasserstoff, Alkyl, z.B. C₁-₄₋Alkyl, Cycloalkyl, z.B. C₅₋₇-Cycloalkyl, oder Aryl, z.B. Phenyl, sind;
Q SiR⁶R⁷, GeR⁶R⁷, SnR⁶R⁷, AsR⁶, S, SO₂ oder Se ist;
R⁶ und R⁷ unabhängig voneinander Alkyl, z.B. C₁-₄₋Alkyl, Cycloalkyl, z.B. C₅₋₇-Cycloalkyl oder Aryl, z.B. Phenyl, sind; oder R₆ und R₇ jeweils eine bivalente Alkylengruppe sind und zusammen mit einem Si-, Ge- oder Sn-Atom einen Ring, insbesondere einen 4- bis 7-gliedrigen Ring bilden.

Ein weiterer Aspekt der Erfindung ist ein Metallkomplex, insbesondere ein Palladium- oder Nickelkomplex,
der ein Phosphin der Formel **(1)** als Ligand L¹ enthält.

Ein weiterer Aspekt der Erfindung ist eine Kombination, enthaltend
- eine Metallverbindung, insbesondere eine Palladium- oder Nickelverbindung und
- ein Phosphin der Formel **(1).**

Ein weiterer Aspekt der Erfindung ist die Verwendung eines Metallkomplexes, insbesondere eines Palladium- oder Nickelkomplexes, der ein Phosphin der Formel **(1)** als Ligand enthält, als Katalysator, insbesondere als Katalysator für eine Kupplungsreaktion, insbesondere für eine Kreuzkupplungsreaktion.

Noch ein weiterer Aspekt der Erfindung ist ein Verfahren zur Durchführung einer Kupplungsreaktion, wobei die Edukte der Kupplungsreaktion in Gegenwart eines erfindungsgemäßen Metallkomplexes, insbesondere eines Palladium- oder Nickelkomplexes, der ein Phosphin der Formel (1) enthält, oder einer erfindungsgemäßen Kombination umgesetzt werden und das Produkt der Kupplungsreaktion gegebenenfalls isoliert wird.

Der Begriff Halogen im Sinne der vorliegenden Erfindung bedeutet F, Cl, Br oder I. Vorzugsweise bedeutet Halogen Br oder I.

Der Begriff Alkyl, insbesondere für einen der Reste R¹, R², R³, R⁴, R⁵, R⁶ und R⁷ bedeutet ein geradkettiges oder verzweigtes, unsubstituiertes oder substituiertes C₁-C₁₀ Alkyl, wobei R¹, R², R⁶ und R⁷ vorzugsweise ein C₁₋₈ Alkyl, R³ und R⁴ vorzugsweise ein C₁₋₄ Alkyl und R⁵ vorzugsweise ein C₁₋₅ Alkyl darstellen, wobei jedes Alkyl einen oder mehrere Substituenten unabhängig ausgewählt aus Halogen, Amin, Cycloalkyl, Aryl, O-Alkyl, O-Cycloalkyl oder O-Aryl tragen kann.

Der Begriff Cycloalkyl, insbesondere für einen der Reste R¹, R², R³, R⁴, R⁵, R⁶ und R⁷ bedeutet ein C₃-₈ Cycloalkyl, wobei R¹, R², R⁶ und R⁷ vorzugsweise ein C₃₋₈ Cycloalkyl, R³ und R⁴ vorzugsweise ein C₃₋₅ Cycloalkyl und R⁵ vorzugsweise ein C₃₋₆ Cycloalkyl darstellen, wobei jedes Cycloalkyl einen oder mehrere Substituenten unabhängig ausgewählt aus Halogen, Amin, Alkyl, O-Alkyl, oder O-Cycloalkyl, oder O-Aryl tragen kann. In bestimmten Ausführungsformen kann Cycloalkyl auch ein heterocyclisches Ringsystem mit einem oder mehreren Heteroatome im Ring, z.B. N, O oder S, darstellen. In weiteren Ausführungsformen bedeutet Cycloalkyl ein carbocyclisches Ringsystem.

Der Begriff Aryl, insbesondere für einen der Reste R¹, R², R³, R⁴, R⁵, R⁶ und R⁷ bedeutet C₅-C₁₄ Aryl oder C₆-C₁₄ Aryl, vorzugsweise Phenyl oder Naphthyl und besonders bevorzugt Phenyl, wobei jedes Aryl einen oder mehrere Substituenten unabhängig ausgewählt aus Halogen, Alkyl, O-Alkyl, Cycloalkyl, O-Cycloalkyl, Aryl oder O-Aryl tragen kann. In bestimmten Ausführungsformen kann Aryl auch ein heterocyclisches Ringsystem mit einem oder mehreren Heteroatome im Ring, z.B. N, O oder S, darstellen. In weiteren Ausführungsformen bedeutet Aryl ein carbocyclisches Ringsystem.

Die cyclischen Biarylphosphine der vorliegenden Erfindung enthalten in dem heterocyclischen Ring gemäß Formel (1) neben dem Phosphoratom noch ein weiteres Heteroatom als Ringatom, wobei dieses zusätzliche Heteroringatom aus der Gruppe bestehend aus Si, Ge, Sn, As oder Se ausgewählt ist.

Das Phosphin der Formel (1) enthält p Reste OR¹ und q Reste OR², wobei p 0, 1, 2, 3 oder 4 ist, und q 1, 2, 3, 4, oder 5 ist. Die Summe von p + q beträgt mindestens 1. Vorzugsweise beträgt die Summe p + q mindestens 2. Besonders bevorzugt ist p 1, 2, 3 oder 4 und/oder q ist 1, 2, 3, 4 oder 5.

OR¹ und OR² sind vorzugsweise ausgewählt aus O-Methyl, O-Phenyl, O-iso-Propyl und O-tert-Butyl.

R³, R⁴ und R⁵ sind vorzugsweise ausgewählt aus H, Methyl und Trimethylsilyl.

Q ist vorzugsweise SiR⁶R⁷. R⁶ und R⁷ sind vorzugsweise jeweils unabhängig ausgewählt aus Methyl, Phenyl, das optional mit einer oder mehreren C₁₋₆-Alkyl-, O-C₁₋₆-Alkyl- oder O-C₁₋₆-Arylgruppen substituiert ist.

Wie nachfolgend noch eingehender beschrieben wird, ermöglicht die Verwendung des erfindungsgemäßen Phosphins als Ligand in Metallkomplexen, z.B. in Pd-Komplexen, die Durchführung einer C-C-Kreuzkupplungsreaktion oder einer C-N-Kreuzkupplungsreaktion, beispielsweise der Buchwald-Hartwig-Kupplung, die bei relativ niedrigen Reaktionstemperaturen und relativ kurzen Reaktionszeiten zu hohen Ausbeuten führt, selbst wenn ein N-heterocyclisches Arylhalogenid oder -pseudohalogenid als Reaktant eingesetzt wird.

Der Metallkomplex, z.B. Palladium- oder Nickelkomplex, der das erfindungsgemäße Phosphin als einen Liganden enthält, kann bereits vor der zu katalysierenden Reaktion hergestellt und gegebenenfalls bis zu seiner Verwendung gelagert werden. Alternativ ist es auch möglich, dass das erfindungsgemäße Phosphin und eine als Präkursor fungierende Metallverbindung, z.B. Palladium- oder Nickelverbindung dem Reaktionsmedium zugegeben werden, so dass die Bildung eines Metallkomplexes, z.B. Palladium- oder Nickelkomplexes, der das erfindungsgemäße Phosphin als einen Liganden enthält, *in situ* im Reaktionsmedium der Kupplungsreaktion erfolgt.

Der erfindungsgemäße Metallkomplex, z.B. Palladium- oder Nickelkomplex, der als einen Liganden L¹ das oben beschriebene erfindungsgemäße Phosphin enthält., kann zusätzlich noch einen oder mehrere, z.B. 1, 2 oder 3 Liganden L² enthalten, die jeweils kein erfindungsgemäßes Phosphin sind. Geeignete Liganden L² für Metallkomplexe, z.B. Palladium- oder Nickelkomplexe sind dem Fachmann bekannt.

Beispielsweise sind die weiteren Liganden L² des erfindungsgemäßen Metallkomplexes, z.B. Pd-Komplexes unabhängig voneinander ausgewählt aus einem Halogenid (z.B. Cl⁻, Br⁻ oder I⁻); einem Aryl (z.B. Phenyl), einem Nitril (z.B. Acetonitril, Propionitril oder Benzonitril); einem Carboxylat (z.B. Acetat); einem konjugierten Dienon (z.B. ein 1,4-Dien-3-on wie Dibenzylidenaceton (dba)); einem Phosphin, das kein erfindungsgemäßes Phosphin ist (z.B. ein nicht-cyclisches Phosphin); einem Pseudohalogenid (z.B. CN⁻ oder OCN⁻) einem Amin oder Acetylacetonat.

Der Ligand L², der kein erfindungsgemäßes Phosphin ist, kann ein einzähniger oder alternativ ein mehrzähniger Ligand sein. Beispiele für solche mehrzähnigen Liganden umfassen Arylalkylamine oder Arylamine (z.B. Phenethylamin oder Naphthylamin) und Monoanionen davon.

Sofern der Ligand L² ein Phosphin ist, handelt es sich bevorzugt um ein nicht-cyclisches Phosphin, d.h. ein Phosphin, das keinen phosphorhaltigen Ring aufweist. Geeignete nicht-cyclische Phosphinliganden für Metall- bzw. Palladiumkomplexe sind dem Fachmann bekannt.

Bei dem Phosphinliganden L², der kein erfindungsgemäßes Phosphin ist, handelt es sich beispielsweise um ein Tri-C₁₋₆-Alkylphosphin, ein Tri-C₅₋₇-Cycloalkylphosphin oder ein Triarylphosphin (insbesondere ein Triphenylphosphin), wobei jede der Arylgruppen (die bevorzugt Phenylgruppen sind) optional durch eine oder mehrere C₁₋₄-Haloalkylgruppen (z.B. -CF₃), C₁₋₄-Alkylgruppen (z.B. Methyl) und/oder C₁₋₄ -Alkoxygruppen (z.B. Methoxy) substituiert ist.

Beispielsweise weist der Phosphinligand L² eine der folgenden Formeln **(2), (3)** oder **(4)** auf:

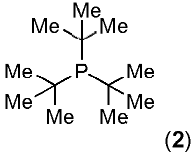

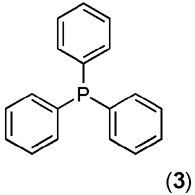

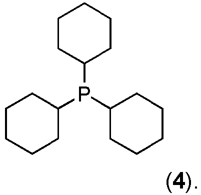

Alternativ kann es sich bei dem Phosphinliganden L² um ein Phosphin der folgenden Formel (5) handeln:

P(R¹)(R²)(R³) **(5)**

wobei R¹ und R² unabhängig voneinander ausgewählt sind aus C₁₋₆-Alkyl and C₅₋₇-Cycloalkyl, z.B. Cyclohexyl, R³ Biphenyl ist, das optional durch eine oder mehrere C₁₋₆-Alkyl- oder C₁₋₆-Haloalkylgruppen, C₁₋₆-Alkoxy- oder C₁₋₆-Haloalkoxygruppen, Phenylgruppen oder Pyridylgruppen substituiert ist.

Beispielsweise weist der Phosphinligand L² eine der folgenden Formeln **(6), (7), (8)** oder **(9)** auf:

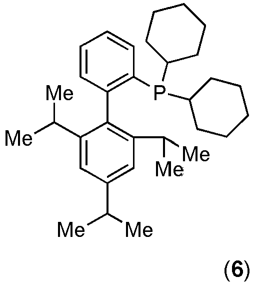

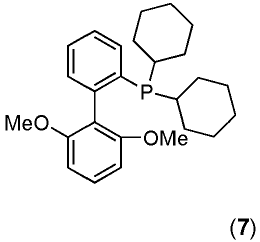

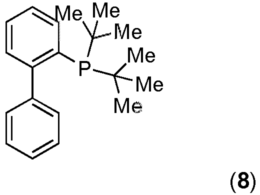

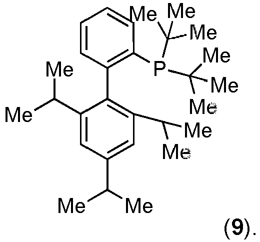

Ein erfindungsgemäßer Palladiumkomplex weist beispielsweise die folgende Formel **(10)** auf: 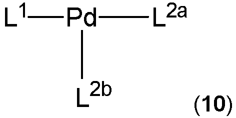 wobei
L¹ ein erfindungsgemäßes Phosphin der Formel **(1)** ist, und
L^{2a} und L^{2b} jeweils ein Ligand ist, der kein erfindungsgemäßes Phosphin ist.

Hinsichtlich geeigneter Liganden L^{2a} und L^{2b} kann auf die obigen Ausführungen zu dem Liganden L² verwiesen werden. Beispielsweise sind die Liganden L^{2a} und L^{2b} des erfindungsgemäßen Pd-Komplexes unabhängig voneinander ausgewählt aus einem Halogenid, z.B. Cl⁻, Br⁻ oder I⁻; einem Aryl; einem Nitril (z.B. Acetonitril, Propionitril oder Benzonitril); einem Carboxylat (z.B. Acetat); einem konjugierten Dienon, z.B. ein 1,4-Dien-3-on wie Dibenzylidenaceton (dba); einem Phosphin, das kein erfindungsgemäßes Phosphin ist (z.B. ein nicht-cyclisches Phosphin); einem Pseudohalogenid (z.B. CN⁻ oder OCN⁻) einem Amin oder Acetylacetonat.

Ein beispielhafter Palladiumkomplex der vorliegenden Erfindung weist die folgende Formel **(11)** auf: 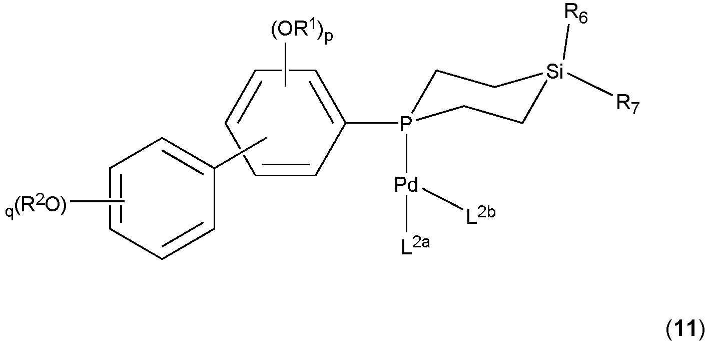 wobei p, q, R¹, R², R⁶ und R⁷ sowie die Liganden L^{2a} und L^{2b} die oben angegebenen Bedeutungen aufweisen.

Beispielsweise ist der Ligand L^{2a} Dibenzylidenaceton (dba) oder Acetonitril und der Ligand L^{2b} wird aus einem der oben für L² angegebenen Liganden ausgewählt.

Ein weiterer beispielhafter Palladiumkomplex der vorliegenden Erfindung weist die folgende Formel **(12)** auf: 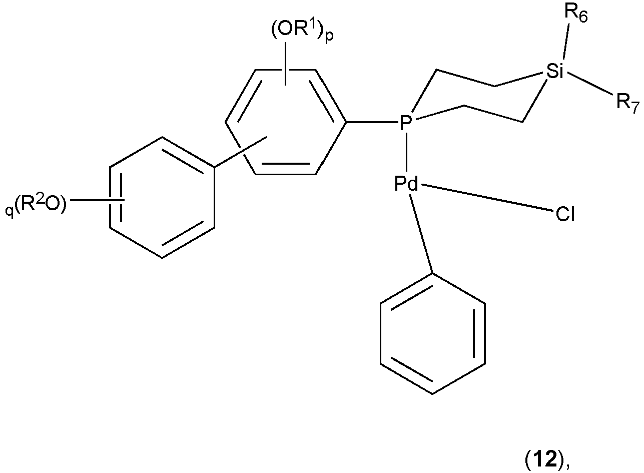 wobei p, q, R¹, R², R⁶ und R⁷ die oben angegebenen Bedeutungen aufweisen.

Ein weiterer beispielhafter Palladiumkomplex der vorliegenden Erfindung weist die folgende Formel **(13)** auf: 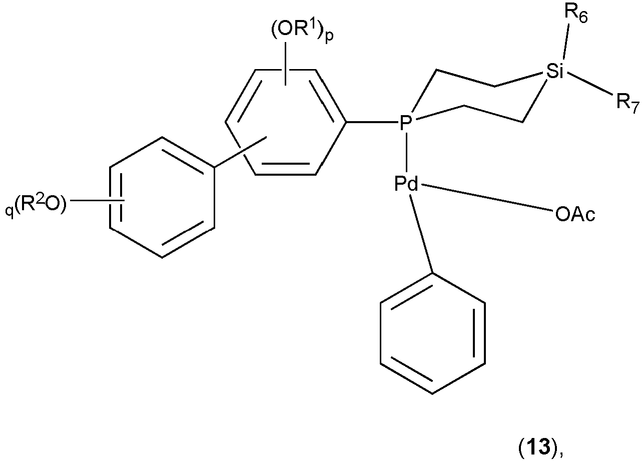 wobei p, q, R¹, R², R⁶ und R⁷ die oben angegebenen Bedeutungen aufweisen.

Wie oben bereits erwähnt, können das erfindungsgemäße Phosphin und eine als Präkursor fungierende Metall- bzw. Palladiumverbindung, die das erfindungsgemäße Phosphin noch nicht enthält, dem Reaktionsmedium zugegeben werden, so dass die Bildung eines Metall- bzw. Palladiumkomplexes, der das erfindungsgemäße Phosphin als einen Liganden enthält, *in-situ* im Reaktionsmedium der Kupplungsreaktion erfolgt.

Die vorliegende Erfindung betrifft daher auch eine Kombination, enthaltend
- eine Metall- bzw. Palladium- oder Nickelverbindung und
- das oben beschriebene erfindungsgemäße Phosphin.

Die Metall- bzw. Palladium- oder Nickelverbindung der erfindungsgemäßen Kombination enthält üblicherweise kein erfindungsgemäßes Phosphin.

Die Kombination kann in einem Behälter vorliegen, der sowohl die Metall- bzw. Palladium- oder Nickelverbindung als auch das erfindungsgemäße Phosphin enthält. Optional kann die Kombination in Form eines Kits, der die Metall- bzw. Palladium- oder Nickelverbindung und das erfindungsgemäße Phosphin in getrennten Behältern enthält, vorliegen.

Die Metall- bzw. Palladiumverbindung ist beispielsweise ein Metall- bzw. Palladiumsalz oder ein Metall- bzw. Palladiumkomplex, dessen Liganden kein erfindungsgemäßes Phosphin sind.

Das Metall- bzw. Palladiumsalz ist beispielsweise ein Palladiumacetat, ein Palladiumhalogenid (z.B. ein Palladiumchlorid, Palladiumbromid oder Palladiumiodid) oder ein Palladiumpseudohalogenid oder ein Gemisch aus mindestens zwei dieser Salze.

Handelt es sich bei der Metall- bzw. Palladiumverbindung um einen Metall- bzw. Palladiumkomplex, so sind dessen Liganden unabhängig voneinander beispielsweise ausgewählt aus einem Halogenid (z.B. Cl⁻, Br⁻ oder I⁻); einem Phosphin, das kein erfindungsgemäßes Phosphin ist (z.B. ein nicht-cyclisches Phosphin); einem konjugierten Dienon, z.B. ein 1,4-Dien-3-on wie Dibenzylidenaceton (dba); einem Nitril, z.B. Acetonitril, Propionitril oder Benzonitril; einem Acetylacetonat; einem Carboxylat, z.B. Acetat; einem Pseudohalogenid, z.B. CN⁻ oder OCN⁻, einem Amin oder einem Aryl.

Hinsichtlich des Phosphinliganden, der kein erfindungsgemäßes Phosphin ist, kann auf die obigen Ausführungen zu Ligand L² verwiesen werden. Bevorzugt ist der Phosphinligand, der kein erfindungsgemäßes Phosphin ist, ein nicht-cyclisches Phosphin, d.h. ein Phosphin, das keinen phosphorhaltigen Ring aufweist. Geeignete nicht-cyclische Phosphinliganden für Palladiumkomplexe sind dem Fachmann bekannt. Bei dem Phosphinliganden, der kein erfindungsgemäßes Phosphin ist, handelt es sich beispielsweise um ein Tri-C₁₋₆-Alkylphosphin, ein Tri-C₅₋₇-Cycloalkylphosphin oder ein Triarylphosphin, insbesondere ein Triphenylphosphin, wobei jede der Arylgruppen, die bevorzugt Phenylgruppen sind, optional durch eine oder mehrere C₁₋₄-Haloalkylgruppen (z.B. -CF₃), C₁₋₄-Alkylgruppen (z.B. Methyl) oder C₁₋₄ -Alkoxygruppen (z.B. Methoxy) substituiert ist. Alternativ kann es sich bei dem Phosphinliganden, der kein erfindungsgemäßes Phosphin ist, beispielsweise um ein Phosphin der oben beschriebenen Formel (8) handeln.

Als beispielhafte Palladiumverbindungen der erfindungsgemäßen Kombination können Folgende genannt werden: ein Palladiumdibenzyliden-Komplex, z.B. Pd₂(dba)₃ oder Pd(dba)₂); PdCl₂(PR₃)₂, wobei R ein Phenyl ist, das optional mit einer oder mehreren C₁₋₆-Alkylgruppen substituiert ist, ein C₅₋₇-Cycloalkyl oder ein C₁₋₆-Alkyl ist; ein Palladiumacetat, z.B. Pd₂(OAc)₃); ein Palladiumacetylacetonat; ein PdX₂, wobei X ein Halogenid oder Pseudohalogenid ist; ein Pd(RCN)₂Cl₂, wobei R ein Phenyl oder Methyl ist.

Die vorliegende Erfindung betrifft außerdem die Verwendung des oben beschriebenen erfindungsgemäßen Metallkomplexes, z.B. Palladium- oder Nickelkomplexes, oder der oben beschriebenen erfindungsgemäßen Kombination als Katalysator in einer Kreuzkupplungsreaktion.

Die Kreuzkupplungsreaktion ist beispielsweise eine C-C- oder C-N-Kreuzkupplungsreaktion. Eine bevorzugte C-N-Kreuzkupplungsreaktion ist die Buchwald-Hartwig-Kupplung. Wie dem Fachmann bekannt ist, handelt es sich bei der Buchwald-Hartwig-Kupplung um eine Kupplungsreaktion, bei der ein Aryl- oder Heteroarylhalogenid, -pseudohalogenid oder - sulfonat und ein primäres oder sekundäres Amin in Anwesenheit eines palladiumhaltigen Katalysators (und bevorzugt einer Base) unter Ausbildung einer C-N-Bindung miteinander umgesetzt werden.

Die C-C-Kreuzkupplungsreaktion ist beispielsweise eine Suzuki-Miyaura-Kupplung. Wie dem Fachmann bekannt ist, handelt es sich bei der Suzuki-Miyaura-Kupplung um eine Kupplungsreaktion, bei der eine Organobor-Verbindung und beispielsweise ein Aryl- oder Heteroarylhalogenid, -pseudohalogenid oder -sulfonat in Anwesenheit eines Katalysators, z.B. eines palladiumhaltigen Katalysators, unter Ausbildung einer C-C-Bindung miteinander umgesetzt werden.

Die vorliegende Erfindung betrifft außerdem ein Verfahren zur Herstellung eines Aryl- oder Heteroarylamins, wobei eine Verbindung der Formel **(14)**

Ar-X **(14)**

wobei
Ar ein Aryl oder Heteroaryl ist,
X ein Halogenatom, eine Sulfonatgruppe (z.B. Trifluormethansulfonat-O-Tf) oder eine Pseudohalogengruppe (z.B. -CN, -OCN oder -NCO) ist,
mit einem primären oder sekundären Amin in Anwesenheit des oben beschriebenen erfindungsgemäßen Metallokomplexes, z.B. Palladium- oder Nickelkomplexes, oder der oben beschriebenen erfindungsgemäßen Kombination umgesetzt wird.

Geeignete Reaktionsbedingungen für die Buchwald-Hartwig-Kupplung sind dem Fachmann bekannt. Bevorzugt erfolgt die Umsetzung in Anwesenheit einer Base.

Im Folgenden soll die Erfindung durch die nachfolgenden Beispiele näher erläutert werden.

### Beispiele

### Herstellung eines erfindungsgemäßen cyclischen Biarylphosphins

Ein erfindungsgemäßes Biaryl-Phosphasilinan der folgenden Formel (15): 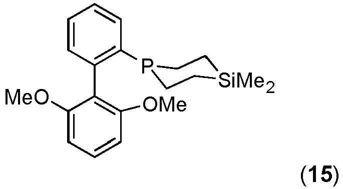 wurde nach dem folgenden Reaktionsschema hergestellt:

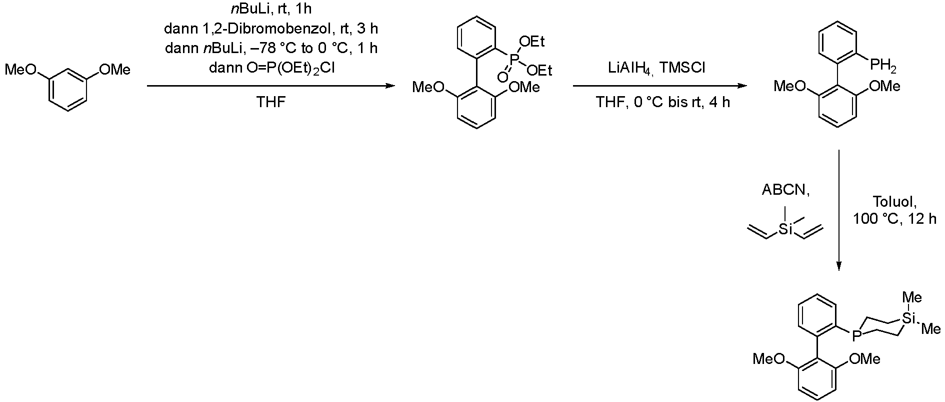

Chemischer Name des cyclischen Biarylphosphins der Formel **(15):**
4,4-Dimethyl-1-(2',,6'-dimethoxy-[1,1-biphenyl]-2-yl)-1,4-phosphasilinan

### 1.1 Diethyl (2',6'-dimethoxy-[1,1'-biphenyl]-2-yl)phosphonat:

1,3-Dimethoxybenzol (5.9 mL, 45 mmol, 1.2 equiv.) wurde in THF (150 mL) gelöst. *n*BuLi (2.5 M in Hexan, 18 mL, 45 mmol, 1.2 equiv.) wurde tropfenweise bei RT (Raumtemperatur) hinzugefügt und anschließend für eine Stunde gerührt. 1,2-Dibromobenzol (4.5 mL, 38 mmol, 1.0 equiv.) wurde langsam zugetropft und die Lösung wurde vier Stunden bei RT gerührt. THF (150 mL) und TMEDA (Tetramethylethylendiamin) (6.1 mL, 41 mmol, 1.1 equiv.) wurden der Lösung zugefügt, anschließend wurde die Lösung auf -78 °C gekühlt. *n*BuLi (2.5 M in Hexan, 16 mL, 41 mmol, 1.1 equiv.) wurde zugetropft und die Lösung wurde bei 0 °C gerührt. Die Lösung wurde wieder auf -78 °C gekühlt und Diethylchlorphosphat (6.5 mL, 45 mmol, 1.2 equiv.) wurde zugetropft. Die Lösung wurde sehr langsam über Nacht auf Raumtemperatur erwärmt und insgesamt für zwölf Stunden gerührt. Die Reaktionslösung wurde mit wässriger, gesättigter NH₄Cl-Lösung (150 mL) versetzt und mit EtOAc (3 × 150 mL) extrahiert. Die vereinigten, organischen Phasen wurden mit wässriger, gesättigter NaCl-Lösung (50 mL) gewaschen und über Na₂SO₄ getrocknet. Das Lösemittel wurde unter vermindertem Druck entfernt und der Rohprodukt wurde in EtOAc umkristallisiert, um Diethyl (2',6'-dimethoxy-[1,1'-biphenyl]-2-yl)phosphonat (9.01 g, 25.7 mmol, 69%) als farblosen Feststoff zu erhalten.

**¹H NMR** (400 MHz, CDCl₃) δ [ppm] = 8.07 (dddd, *J* = 14.2, 7.7, 1.5, 0.6 Hz, 1H), 7.57 (tt, *J =* 7.5, 1.5 Hz, 1H), 7.42 (tdd, *J* = 7.6, 3.7, 1.3 Hz, 1H), 7.30 (t, *J =* 8.4 Hz, 1H), 7.21 (dddd, *J =* 7.7, 5.3, 1.3, 0.6 Hz, 1H), 6.60 (d, *J =* 8.4 Hz, 2H), 4.12 (q, *J =* 7.1 Hz, 1H), 3.98 - 3.73 (m, 4H), 3.69 (s, 6H), 1.20 - 1.11 (m, 6H).

**¹³C{¹H} NMR** (101 MHz, CDCl₃) δ [ppm] = 158.1, 139.06 (*J* = 7.4 Hz), 134.2 (*J* = 10.4 Hz), 132.1 (*J* = 1.0 Hz), 132.0 (*J* = 10.4 Hz), 129.3,128.6 (J = 186.4 Hz), 127.7, 126.9 (J = 15.3 Hz), 118.6 (J = 4.3, 103.4, 61.6 (J = 5.6 Hz), 55.7, 16.4 (J = 6.8 Hz).

**³¹P{¹H} NMR** (162 MHz, CDCl₃) δ [ppm] = 18.2.

### 1.2 (2',6'-Dimethoxy-[1,1'-biphenyl]-2-yl)phosphan:

Diethyl(2',6'-dimethoxy-[1,1'-biphenyl]-2-yl)phosphonat (3.50 g, 10.0 mmol, 1.0 equiv.) wurde in THF (50 mL) gelöst und auf 0 °C gekühlt. LiAlH₄-Suspension (2 M in THF, 13 mL, 26 mmol, 2.5 equiv.) wurde in einem weiteren Kolben auf 0 °C gekühlt und TMSCI (Chlor(trimethyl)silan) (3.8 mL, 4.8 mmol, 2.5 equiv.) wurde tropfenweise hinzugefügt. Diese Lösung wurde 30 min bei 0 °C gerührt und anschließend wurde langsam die Phosphonat-Lösung zugetropft (Vorsicht: Gasentwicklung). Die Reaktionsmischung wurde zwölf Stunden bei Raumtemperatur gerührt und anschließend auf 0 °C gekühlt und mit entgastem EtOAc (100 mL), gefolgt von entgaster, wässriger HCl-Lösung (1 M, 100 mL) gequencht. Nach 30 min Rühren wurde die Lösung mit EtOAc (2×100 mL) extrahiert und die vereinigten organischen Phasen wurden mit gesättigter NaCl-Lösung gewaschen, über Na₂SO₄ getrocknet und unter vermindertem Druck wurde das Lösemittel entfernt, um (2',6'-Dimethoxy-[1,1'-biphenyl]-2-yl)phosphan (2.45 g, 9.80 mmol, 98%) als farblosen Feststoff zu erhalten.

**¹H NMR** (400 MHz, CDCl₃) δ [ppm] = 7.71 - 7.60 (m, 1H), 7.44 - 7.32 (m, 2H), 7.32 - 7.25 (m, 1H), 7.22 (ddd, *J* = 7.5, 2.8, 1.4 Hz, 1H), 6.68 (d, *J* = 8.4 Hz, 2H), 3.75 (s, 6H), 3.67 (d, *J* = 203.4 Hz. 2H).

**¹³C{¹H} NMR** (101 MHz, CDCl₃) δ [ppm] = 157.6, 139.3 (d, *J* = 14.7 Hz), 135.0 (d, *J =* 10.6 Hz), 131.0 (d, *J =* 3.0 Hz), 130.6 (d, *J =* 6.9 Hz), 129.4, 128.1, 127.2 (d, *J =* 4.3 Hz), 119.2 (d, *J =* 3.6 Hz), 104.1, 56.0.

**³¹P{¹H} NMR** (162 MHz, CDCl₃) δ [ppm] = -128.3.

### 1.3 1-(2',6'-Dimethoxy-[1,1'-biphenyl]-2-yl)-4,4-dimethyl-1,4-phosphasilinan:

Die Reaktion wurde in einem 10 mL-Young-Schlenkrohr durchgeführt. (2',6'-Dimethoxy-[1,1'-biphenyl]-2-yl)phosphan (246 mg, 1.00 mmol, 1.00 equiv.) und ABCN (24.4 mg, 100 µmol, 10 mol%) wurden in Toluol (10 mL) gelöst. Dimethyldivinylsilan (0.21 mL, 1.4 mmol, 1.4 equiv.) wurde zugefügt, das Schlenkrohr wurde verschlossen und die Reaktion wurde bei 100 °C für zwölf Stunden gerührt. Säulenchromatographie lieferte 1-(2',6'-Dimethoxy-[1,1'-biphenyl]-2-yl)-4,4-dimethyl-1,4-phosphasilinan (200 mg, 0.560 mmol, 56%) als farblosen Feststoff.

**¹H NMR** (400 MHz, C₆D₆) δ [ppm] = 7.63 (ddd, J = 7.7, 3.3, 1.9 Hz, 1H), 7.40 - 7.34 (m), 7.28 - 7.23 (m), 7.23 - 7.20 (m), 7.20 - 7.16 (m), 6.44 (d, J = 8.3 Hz, 2H), 3.30 (s, 6H), 2.07 - 1.78 (m, 4H), 0.99 - 0.71 (m, 4H), -0.01 (s, 3H), -0.16 (s, 3H).

**¹³C{¹H} NMR** (101 MHz, C₆D₆) δ [ppm] = 158.5, 141.1(d, *J* = 6.4 Hz), 141.1(d, *J* = 6.4 Hz,), 140.9 (d, *J =* 7.1 Hz), 131.8 (d, *J =* 5.3 Hz), 130.2, 129.1, 127.9, 127.4, 120.4 (d, *J =* 6.2 Hz), 104.1, 55.2, 24.0 (d, *J =* 13.7 Hz), 12.0 (d, *J =* 16.6 Hz), -2.2, -4.0.

**³¹P{¹H} NMR** (162 MHz, C₆D₆) δ [ppm] = -30.33.

**HRMS (EI)** m/z: calculated for C₂₀H₂₆O₂PSi [M-H]⁺:357.1434, found: 357.1430.

### Verwendung von Zusammensetzungen, die ein Biarylphosphin und eine Palladiumverbindung enthalten, als Katalysator in einer Buchwald-Hartwig-Kupplung

In dem nachfolgend beschriebenen erfindungsgemäßen Beispiel 1 wurde das cyclische Biarylphosphin der Formel **(15)** verwendet, d.h.:

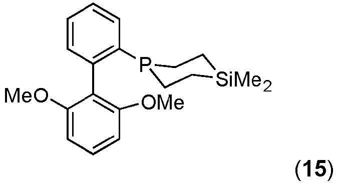

In dem Vergleichsbeispiel 1 wurde ein nicht-cyclisches Biarylphosphin der folgenden Formel (16) verwendet:

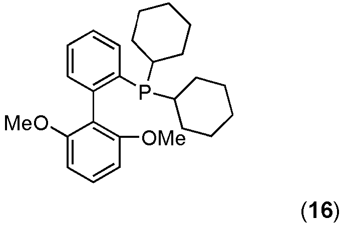

Als Palladiumverbindung wurde in allen Beispielen (d.h. dem erfindungsgemäßen Beispiel 1 und dem Vergleichsbeispiel 1 ein Palladiumdibenzylidenkomplex (Pd₂(dba)₃) verwendet. Diese Palladiumverbindung und das Phosphin der Formel **(15)** oder **(16)** wurden dem Reaktionsmedium zugegeben, so dass sich *in-situ* ein Palladiumkomplex, der das Phosphin als einen seiner Liganden enthält, bilden konnte.

### Beispiel 1:

In Beispiel 1 wurden 2-Chlorchinolin **(17)** und Piperidin **(18)** als Reaktanten für die Buchwald-Hartwig Kupplung verwendet.

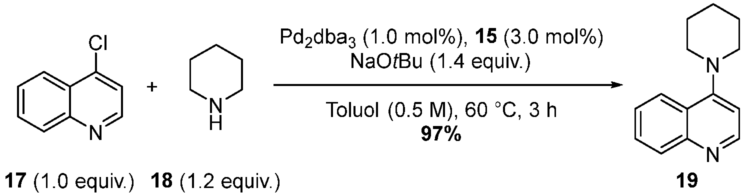

4-Chlorchinolin (39 µL, 0.30 mmol, 1.0 equiv.), Pd₂dba₃ (2.8 mg, 3.0 µmol, 1 mol%.), 1-(2',6'-Dimethoxy-[1,1'-biphenyl]-2-yl)-4,4-dimethyl-1,4-phosphasilinan **(15)** (3.2 mg, 9.0 µmol, 3 mol%.) und NaOtBu (40.4 mg, 0.420 mmol, 1.40 equiv) wurden in Toluol (0.6 mL) aufgenommen. Die Lösung wurde bei 60 °C für 15 min gerührt. Piperidin (36 µL, 0.36 mmol, 1.2 equiv.) wurde zugegeben und die Reaktion wurde bei 60 °C für drei Stunden gerührt. Die Lösung wurde mit H₂O (5 mL) versetzt und mit AcOEt (3x5 mL) extrahiert. Die vereinigten organischen Phasen wurden mit gesättigter NaCl-Lösung gewaschen, über Na₂SO₄ getrocknet und unter vermindertem Druck eingeengt. Säulenchromatografie lieferte das gewünschte Produkt **(19)** (61.7 mg, 0.290 mmol, 97%) als farbloses Öl in einer fast quantitativen Ausbeute.

**¹H NMR** (400 MHz, CDCl₃) δ [ppm] = 8.69 (d, *J* = 5.1 Hz, 1H), 8.10 - 7.97 (m, 2H), 7.65 (ddd, *J* = 8.4, 6.8, 1.5 Hz, 1H), 7.47 (ddd, *J* = 8.3, 6.8, 1.3 Hz, 1H), 6.82 (d, *J =* 5.1 Hz, 1H), 3.21 (t, *J* = 5.2 Hz, 4H), 1.86 (dq, *J* = 11.0, 5.2 Hz, 4H), 1.71 (q, *J =* 6.0 Hz, 2H).

### Vergleichsbeispiel 1:

In Vergleichsbeispiel 1 waren die Reaktanten und die Synthesebedingungen identisch mit den in Beispiel 1 verwendeten Reaktanten und Synthesebedingungen. Allerdings wurde anstelle des erfindungsgemäßen Phosphins der Formel **(15)** das Phosphin der Formel **(16)** verwendet:

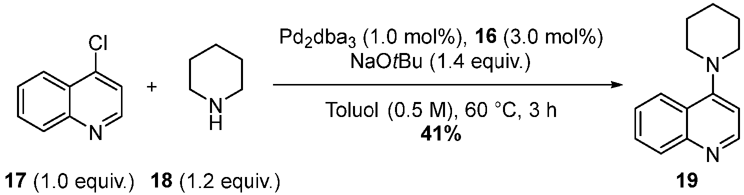

In Vergleichsbeispiel 1 war die Ausbeute des Produkts **(19)** mit 41% ganz erheblich geringer als im erfindungsgemäßen Beispiel 1 mit 97%.

Obwohl in allen Beispielen einer der Reaktanten ein N-heterocyclisches Arylhalogenid war und eine recht kurze Reaktionsdauer bei relativ milder Reaktionstemperatur gewählt wurde, führte die Verwendung des erfindungsgemäßen cyclischen Biarylphosphins als Ligand eines Palladiumkomplexes bei einer palladiumkatalysierten Buchwald-Hartwig-Kupplung zu hohen Produktausbeuten.

### Verwendung einer Zusammensetzung, die ein erfindungsgemäßes cyclisches Biarylphosphin und eine Palladiumverbindung enthält, als Katalysator in einer Suzuki-Miyaura-Kupplung

Unter Verwendung eines erfindungsgemäßen cyclischen Biarylphosphins der Formel **(15)** wurde in dem erfindungsgemäßen Beispiel 2 eine Suzuki-Miyaura-Kupplung mit den im nachfolgenden Reaktionsschema Reaktionsbedingungen durchgeführt. Die Ausbeute ist im Reaktionsschema gegeben.

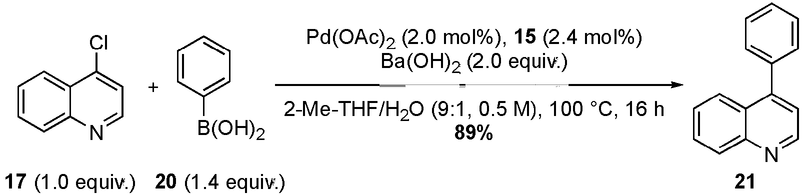

### Beispiel 2:

Phenylboronsäure **(20)** (51 mg, 0.42 mmol, 1.4 equiv), Pd(OAc)₂ (1.4 mg, 6.0 µmol, 2 mol%), 1-(2',6'-Dimethoxy-[1,1'-biphenyl]-2-yl)-4,4-dimethyl-1,4-phosphasilinan **(15)** (2.5 mg, 7.2 µmol, 2.4 mol%) und Ba(OH)₂ (103 mg, 600 µmol, 2.0 equiv) wurden in einer Mischung aus H₂O (0.06 mL) und 2-MeTHF (0.54 mL) gelöst. 4-Chlorchinolin **(17)** (39 µL, 0.30 mmol, 1.0 equiv.) wurde hinzugefügt und die Lösung wurde bei 100 °C für 16 h gerührt. Die Lösemittel wurden *in vacuo* entfernt und der Rückstand wurde säulenchromatografisch gereinigt. Das gewünschte Produkt **(21)** (55.0 mg, 0.268 mmol, 89% Ausbeute) wurde als farbloser Feststoff in einer sehr hohen Ausbeute erhalten.

¹H NMR (400 MHz, CD₂Cl₂) δ [ppm] = 8.93 (d, *J =* 4.4 Hz, 1H), 8.21 - 8.13 (m, 1H), 7.94 (dd, *J* = 8.6, 1.5 Hz, 1H), 7.73 (ddd, *J* = 8.4, 6.8, 1.5 Hz, 1H), 7.58 - 7.47 (m, 6H), 7.35 (d, *J =* 4.4 Hz, 1H).

### Vergleichsbeispiel 2:

In Vergleichsbeispiel 2 waren die Reaktanten und die Synthesebedingungen identisch mit den in Beispiel 2 verwendeten Reaktanten und Synthesebedingungen. Allerdings wurde anstelle des erfindungsgemäßen Phosphins der Formel **(15)** das Phosphin der Formel **(16)** verwendet.

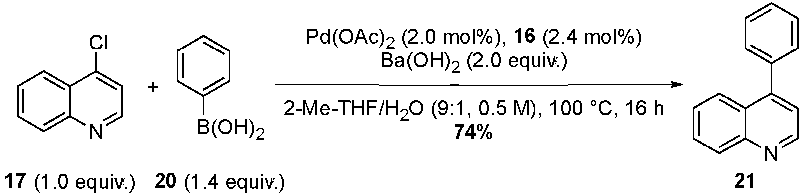

In Vergleichsbeispiel 2 war die Ausbeute des Produkts **(21)** mit 74% erheblich geringer als im erfindungsgemäßen Beispiel 2 mit 89%

Obwohl in beiden Beispielreaktionen und dem jeweiligen Vergleichsbeispiel einer der Reaktanden ein N-heterocyclisches Arylchlorid war und eine recht kurze Reaktionsdauer (4 Std.) bei milder Reaktionstemperatur gewählt wurde, führte die Verwendung des erfindungsgemäßen cyclischen Biarylphosphins als Ligand eines Palladiumkomplexes bei einer palladiumkatalysierten Suzuki-Miyaura-Kupplung zu hohen Produktausbeuten. So wurde auch eine gesteigerte Ausbeute gegenüber den beiden Vergleichsbeispielen erreicht.

## Patentansprüche

1. Phosphin der Formel **(1)** 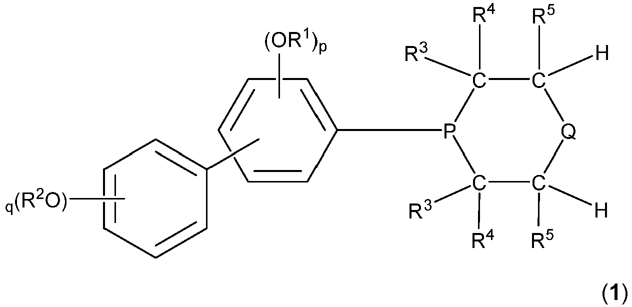 wobei
R¹ jeweils unabhängig Alkyl oder Aryl ist;
R² jeweils unabhängig Alkyl oder Aryl ist;
p 0, 1, 2, 3 oder 4 ist,
q 1, 2, 3, 4, oder 5 ist,
R³, R⁴ und R⁵ unabhängig voneinander Wasserstoff, Alkyl, Cycloalkyl oder Aryl sind;
Q SiR⁶R⁷, GeR⁶R⁷, SnR⁶R⁷, AsR⁶, S, SO₂ oder Se ist;
R⁶ und R⁷ unabhängig voneinander Alkyl, Cycloalkyl, oder Aryl sind; oder R₆ und R₇ jeweils eine bivalente Alkylengruppe sind und zusammen mit einem Si-, Ge- oder Sn-Atom einen Ring bilden.

2. Metallkomplex, der als einen Liganden L¹ das Phosphin gemäß dem Anspruch 1 enthält.

3. Metallkomplex nach Anspruch 2, der ein Palladium- oder Nickelkomplex ist.

4. Metallkomplex nach Anspruch 2 oder 3, der zusätzlich mindestens einen Liganden L² enthält, die jeweils kein Phosphin gemäß dem Anspruch 1 ist.

5. Metallkomplex nach Anspruch 4, wobei der mindestens eine Ligand L² unabhängig voneinander ausgewählt ist aus einem Halogenid; einem Aryl; einem Nitril; einem Carboxylat; einem konjugierten Dienon; einem Phosphin, das kein Phosphin nach Anspruch 1 ist; einem Amin oder Acetylacetonat.

6. Kombination, enthaltend
- eine Metallverbindung, insbesondere eine Palladium- oder Nickelverbindung, und
- ein Phosphin der Formel **(1).**

7. Kombination nach Anspruch 6, wobei die Bestandteile der Kombination gemeinsam in einem Behälter oder separat in mehreren Behältern vorliegen.

8. Kombination nach Anspruch 6 oder 7, wobei die Metallverbindung, insbesondere die Palladium- oder Nickelverbindung, ausgewählt ist aus einem Metallsalz, insbesondere einem Palladium- oder Nickelsalz, einem Metallkomplex, insbesondere einem Palladium- oder Nickelkomplex, der kein Phosphin gemäß Anspruch 1 als Liganden enthält, oder einer Kombination von mehreren Salzen und/oder Komplexen.

9. Kombination nach Anspruch 8, wobei das Metallsalz ein Palladiumsalz ausgewählt aus einem Palladiumacetat, einem Palladiumhalogenid, einem Palladiumpseudohalogenid oder einem Gemisch aus mindestens zwei dieser Salze ist.

10. Kombination nach Anspruch 8, wobei der Metallkomplex, insbesondere der Palladium- oder Nickelkomplex, einen oder mehrere Liganden, unabhängig voneinander ausgewählt aus einem Halogenid; einem Phosphin, das kein Phosphin gemäß Anspruch 1 ist; einem konjugierten Dienon, vorzugsweise einem 1,4-Dien-3-on wie Dibenzylidenaceton; einem Nitril; einem Acetylacetonat; einem Carboxylat; einem Pseudohalogenid; einem Amin; einem Aryl enthält.

11. Verwendung eines Metallkomplexes, insbesondere eines Palladium- oder Nickelkomplexes gemäß einem der Ansprüche 2 - 5 oder einer Kombination gemäß einem der Ansprüche 6 bis 10 als Katalysator, insbesondere als Katalysator in einer Kupplungsreaktion, insbesondere fin einer Kreuzkupplungsreaktion.

12. Verwendung nach Anspruch 11, wobei die Kreuzkupplungsreaktion eine C-C-Kreuzkupplungsreaktion, insbesondere eine Suzuki-Miyaura-Kreuzkupplung, oder eine C-N-Kreuzkupplungsreaktion, insbesondere eine Buchwald-Hartwig-Kupplung ist.

13. Verfahren zur Durchführung einer Kupplungsreaktion, umfassend:
- Umsetzen der Edukte der Kupplungsreaktion in Anwesenheit eines Metallkomplexes, insbesondere eines Palladium- oder eines Nickelkomplexes, gemäß einem der Ansprüche 2 - 5 oder einer Kombination gemäß einem der Ansprüche 6 bis 10, und
- gegebenenfalls Isolieren des Reaktionsprodukts.

14. Verfahren nach Anspruch 13 zur Herstellung eines Aryl- oder Heteroarylamins, wobei eine Verbindung der Formel **(14)**
Ar-X **(14)**
wobei
Ar ein Aryl oder Heteroaryl ist, und
X ein Halogen, ein Sulfonat oder ein Pseudohalogen ist,
mit einem primären oder sekundären Amin umgesetzt wird.
